# EUROPEAN PATENT APPLICATION

(11) **EP 1 245 679 A1**
(43) Date of publication of application: **02.10.2002**
(21) Application number: 00937302.8
(22) Date of filing: 19.06.2000
(51) Int. Cl.: C12N 15/52, C12N 1/21, A61K 39/02, A61K 39/108, A61K 39/112, A61K 39/102

(54) **VACCINE STRAINS AGAINST INFECTION WITH PATHOGENIC BACTERIA**

(30) Priority: 21.12.1999 JP 36301299
(71) Applicant: Japan Science and Technology Corporation, Kawaguchi-shi, Saitama 332-0012 (JP)
(72) Inventor: HIRAGA, Sota, Kyoto 611-0028 (JP)
(74) Representative: Harding, Charles Thomas
(86) International application number: JP0003990
(87) International publication number: WO01046428

(57) **Abstract**

An object of the present invention is to provide a vaccine strain which is extremely safe and has potent antigenicity (immunogenicity), and therefore, can be used as an effective live vaccine against infection with various pathogenic bacteria, and a live vaccine component containing the vaccine strain. For preparation of a vaccine strain against infection with pathogenic bacteria having a double gene mutation and comprised of pathogenic bacteria whose pathogenicity-expressing function is deficient or deteriorated, DNA wherein a mukFEB operon that relates to temperature sensitivity in colony formation has been substituted with a kanamycin-resistant gene is introduced by homologous recombination into a deletion strain of a DNA adenine methylase gene that relates to pathogenicity-expressing function of pathogenic bacteria including pathogenic Escherichia coli, Salmonella typhimurium, Salmonella typhi, Salmonella typhosa, Shigella, Klebsiella, Haemophilus influenzae, Pasteurella and Actinobacillus.

## Description

### Technical Field

The present invention relates to a vaccine strain which is extremely safe and has potent antigenicity (immunogenicity) and therefore, can be used as an effective live vaccine against infection with pathogenic bacteria including pathogenic Escherichia coli, Salmonella typhimurium, Salmonella typhi, Salmonella typhosa, Shigella, Klebsiella, Haemophilus influenzae, Pasteurella and Actinobacillus, and a live vaccine component containing the vaccine strain.

### Background Art

When a living organism is exposed to an antigen, an immune response to the antigen is elicited, and immunological memory is formed as a result of a series of immune responses. When the living organism is exposed to the same antigen for the second time, a quicker and stronger immune response is occurred by the immunological memory. Making use of this property, a pathogen, being attenuated or inactivated in advance, is used to sensitize a living organism for inducing immunological memory. The attenuated or inactivated pathogen is called a vaccine, and roughly classified into four groups: a live vaccine, a killed or inactivated vaccine, a component vaccine (subunit vaccine) and a toxoid.

Among the four kinds of vaccine, a live vaccine has the most potent antigenicity (immunogenicity) , and elicits the most potent immune response in a host, and in general, its toxicity is attenuated or neutralized, so that it can elicit a long-lasting immune response to antigens without triggering the onset of diseases normally related. As a killed vaccine, which is prepared by being inactivated by chemical means or the like that do not inactivate antigenic factors to be presented to an immune system of a host, a pertussis vaccine, a parenteral polio live vaccine and the like are exemplified. As a component vaccine, which contains an immunogenic substance being purified and separated from a microorganism and useful for protection from infection, a hepatitis B vaccine, an influenza vaccine, a herpes virus vaccine, a hepatitis vaccine and the like are exemplified. As a toxoid vaccine, which is prepared by neutralizing a toxin secreted by a pathogen, and is used just like other antigenic factors for stimulating an immune response to the toxin, tetanus toxoid, diphtheria toxoid and the like are exemplified.

Recently, more and more antibiotic-resistant bacteria, in particular, multidrug-resistant bacteria have been found, and expectation has been raised for a safe and effective vaccine against infection with bacteria such as pathogenic Escherichia coli, particularly for a vaccine strain (live vaccine) which has potent antigenicity and excellent durability, and an increasing number of researches and developments for obtaining the vaccine strain has been conducted. For example, it is reported in U. S. A. that a deletion strain of Salmonella typhimurium DNA adenine methylase (dam deletion strain) can be used as an effective vaccine strain whose pathogenicity-expressing function is deficient or deteriorated (Science 284, 967-970, 1999).

Said DNA adenine methylase is known as an enzyme that methylates adenine on a GATC sequence of chromosomal DNA of many species which belong to β group of Proteobacteria including Escherichia coli. For instance, there are about 19,000 GATC sequences in chromosomal DNA of Escherichia coli, and as a replication fork advances from a replication origin, oriC, to two directions, hemimethylated (half-methylated) double-strand DNA wherein a GATC sequence in a nascent strand is not methylated is formed. SeqA is known as a protein which strongly and specifically binds to the hemimethylated GATC sequence (Cell 82, 927-936, 1995). SeqA can bind to hemimethylated DNA to delay methylation caused by DNA adenine methylase, and is thought to be essential to synchronize the initiation of replication of oriC copy in a cell.

Meanwhile, as a temperature-sensitive mutant, a mutant which shows a phenotype different from that of wild type within a certain range of temperature, two types are known. One is a heat-sensitive mutant which shows a phenotype different from that of wild type at a certain temperature or above, and the other is a cold-sensitive mutant which shows a phenotype different from that of wild type at a certain temperature or under. It is generally thought to be often the case that a phenotype of the heat-sensitive mutant becomes heat-sensitive because a protein or RNA, which is a product of a mutated gene, is destabilized or inactivated at a certain temperature or above, and that in the cold-sensitive mutant, as typically observed in a ribosome subunit protein assembly defective (sad) strain, mutation of constituent protein in intracellular structure results in inability to form the structure at a certain temperature or under. In case these mutated genes are essential for growth, proliferation or the like of a cell, the strain becomes a conditional lethal mutant. On the contrary, cold-adaptation mutation, wherein strains are adapted to be able to proliferate at a low temperature in which wild types cannot proliferate, is observed in some bacteria, virus and the like, and these strains are used for the development of a live vaccine against an influenza virus and a polio virus.

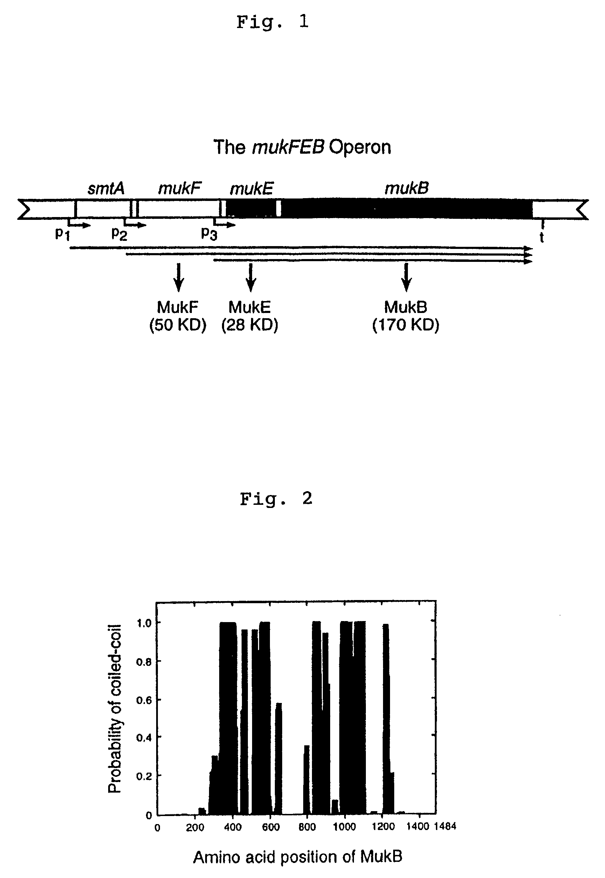

The inventor of the present invention has already isolated a temperature-sensitive mutant of Escherichia coli that spontaneously forms a normal-sized anucleate cell at a low temperature, and reported it (EMBO J. 10, 183-193, 1991). This mutant has a deletion of a gene called mukB in a mukFEB operon (Fig. 1) located at 21 min on a chromosomal map. The mukB gene encodes a protein comprising 1484 amino acid residues (170 kilodalton)(FEMSMicrobiol. Lett. 123, 27-32, 1994), and a MukB protein has an N-terminal globular domain containing a nucleotide binding sequence, a central region containing two α-helical coiled-coil domains, and a C-terminal globular domain being involved in DNA biding ability and ability to bind to MukF and MukE (Fig. 2). Said mukB mutant is not led to death at a low temperature, however, deletion of MukB causes aberrant chromosome partitioning, and the mukB deletion mutant cannot form a colony at a high temperature and many nucleoids are distributed irregularly along elongated cells, suggesting that the MukB protein is essential for chromosome partitioning in Escherichia coli.

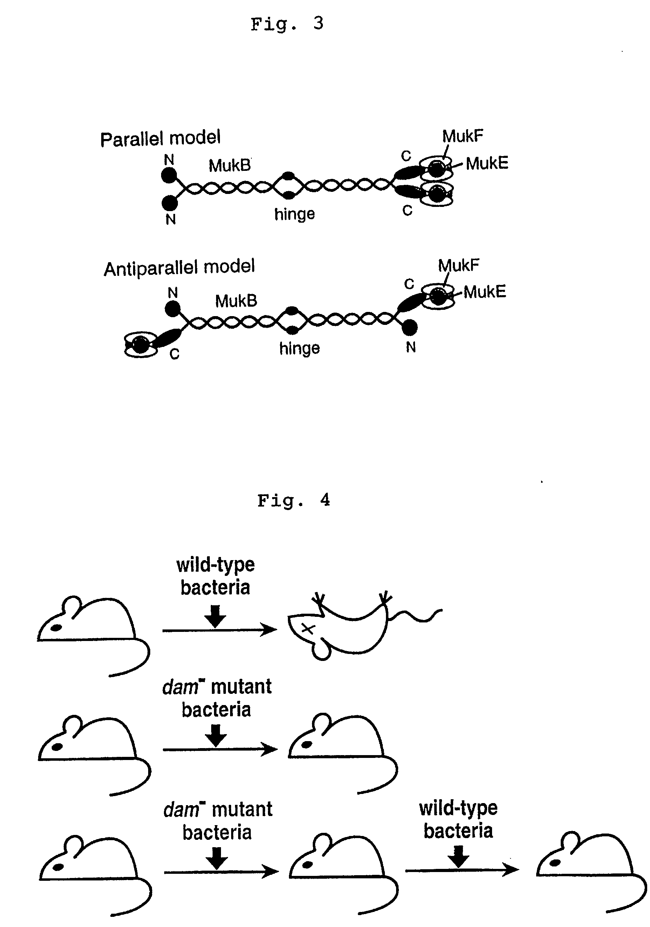

The inventor of the present invention has also found that a deletion mutant of a mukF gene or a mukE gene of a mukFEB operon is a temperature-sensitive mutant in colony formation just like the mukB deletion mutant, and has reported that these proteins are indispensable to chromosome partitioning in Escherichia coli (EMBO J. 10, 183-193, 1991, EMBO J. 11, 5101-5109, 1992, FEMS Microbiol. Lett. 143, 211-216, Mol. Gen. Genet. 250, 241-251, 1996, Annu. Rev. Biochem. 61, 283-306, 1992, EMBO J. 18, 5873-5884, 1999). As to a complex of mukFEB proteins, an expression product of a mukFEB operon, a parallel dimer model and an antiparallel dimer model are considered (Fig. 3). It is known that each deletion mutant of a mukF, a mukE or a mukB gene, and of a mukFEB operon forms colonies at 22 °C, but not above 30°C, and that these muk mutants are defective in normal localization of chromosomes and abnormality in the conformation of its daughter chromosome is observed even at an acceptable temperature of 22° C, and as a result, that these mutants produce anucleate cells with length of normal cells (Mol. Gen. Genet. 250, 241-251, 1996).

As aforementioned, it is reported that a deletion strain of Salmonella typhimurium DNA adenine methylase (dam deletion strain) can be used as an effective vaccine strain whose pathogenicity-expressing function is deficient or deteriorated. However, when a normal dam gene is transferred from other bacterium to this vaccine strain by a conjugational transferable plasmid, a transducing phage or the like in a host cell, there arises a danger that a gene mutation of this vaccine strain is restored to a normal type and the vaccine strain acquires pathogenicity, and it raises a problem in its safety as a vaccine strain. An object of the present invention is to provide a vaccine strain which is extremely safe and has potent antigenicity (immunogenicity), and therefore, can be used as an effective live vaccine against many pathogenic bacteria, and a live vaccine component containing the vaccine strain.

### Disclosure of the Invention

The inventor of the present invention has conducted intensive study to attain the above-mentioned object, and as a result, a double mutant having deletions of a dam gene and a mukFEB operon has been constructed by introducing a mukFEB deletion mutant wherein a mukFEB gene is substituted with an antibiotic-resistant gene into a cell of a deletion mutant of a dam gene related to the pathogenicity-expressing function of a pathogenic bacterium. The double mutant exhibits normal growth in a temperature-independent manner as in the case of the wild type, however, it has been found that when a normal dam gene is transferred and a deleted dam gene is restored to a normal type, the growth arrest or retardation occurs in a temperature-dependent manner, and it leads to death of the mutant. This suggests that this double mutant can be administered to a host organism as a safe vaccine strain because even if reverse mutation occurs, or a DNA adenine methylase gene is transferred from other bacterium by a phage or a conjugational transferable plasmid in the host, the bacterial cell will be led to death. The present invention has completed based on these findings and the like.

The present invention relates to a vaccine strain against infection with pathogenic bacteria being comprised of pathogenic bacteria having at least double gene mutation and whose pathogenicity-expressing function is deficient or deteriorated (claim 1), the vaccine strain against infection with pathogenic bacteria according to claim 1, wherein the pathogenic bacteria having at least double gene mutation and whose pathogenicity-expressing function is deficient or deteriorated exhibit at least the same growth level as that of wild types of the bacteria (claim 2), the vaccine strain against infection with pathogenic bacteria according to claim 1, wherein when one or more gene mutations of the at least double gene mutation are restored to normal types, the pathogenic bacteria are led to death or exhibit growth arrest or retardation (claim 3), the vaccine strain against infection with pathogenic bacteria according to any of claims 1 to 3, wherein at least one of the at least double gene mutation is a mutation of a gene related to the pathogenicity-expressing function of pathogenic bacteria (claim 4), the vaccine strain against infection with pathogenic bacteria according to any of claims 1 to 4, wherein at least one of the at least double gene mutation is a mutation of a gene related to temperature sensitivity of pathogenic bacteria (claim 5), the vaccine strain against infection with pathogenic bacteria according to any of claims 1 to 3, wherein the at least double gene mutation is a double mutation of a gene related to the pathogenicity-expressing function of pathogenic bacteria and of a gene related to temperature sensitivity of pathogenic bacteria (claim 6), the vaccine strain against infection with pathogenic bacteria according to claim 4 or 6, wherein the gene related to the pathogenicity-expressing function is related to a toxin derived from a pathogenic bacterium (claim 7), the vaccine strain against infection with pathogenic bacteria according to claim 4 or 6, wherein the gene related to the pathogenicity-expressing function encodes DNA adenine methylase (claim 8), the vaccine strain against infection with pathogenic bacteria according to claim 5 or 6, wherein the gene related to temperature sensitivity is a mukFEB operon (claim 9), the vaccine strain against infection with pathogenic bacteria according to claim 5 or 6, wherein the gene related to temperature sensitivity is one or more genes selected from a mukF gene, a mukE gene and a mukB gene (claim 10), the vaccine strain against infection with pathogenic bacteria according to any of claims 1 to 3, wherein the at least double gene mutation is a double mutation of a gene encoding DNA adenine methylase and of a mukFEB operon (claim 11), the vaccine strain against infection with pathogenic bacteria according to any of claims 1 to 3, wherein the at least double gene mutation is a double mutation of a gene encoding DNA adenine methylase and of one or more genes selected from a mukF gene, a mukE gene and a mukB gene (claim 12), the vaccine strain against infection with pathogenic bacteria according to any of claims 1 to 3, wherein the at least double gene mutation is a triple mutation of a gene encoding DNA adenine methylase, a gene encoding Seq A, and a mukFEB operon (claim 13), the vaccine strain against infection with pathogenic bacteria according to any of claims 1 to 3, wherein the at least double gene mutation is a triple mutation of a gene encoding DNA adenine methylase, a gene encoding Seq A, and one or more genes selected from a mukF gene, a mukE gene and a mukB gene (claim 14), the vaccine strain against infection with pathogenic bacteria according to any of claims 1 to 14, wherein the gene mutation is one or more gene mutations selected from deletion mutation, frameshift mutation, amino acid substitution mutation, insertional mutation and inversion mutation (claim 15), the vaccine strain against infection with pathogenic bacteria according to any of claims 1 to 15, wherein the pathogenic bacteria invade a cell or tissue of a host (claim 16), the vaccine strain against infection with pathogenic bacteria according to claims 16, wherein the pathogenic bacteria invading a cell or tissue of a host is selected from a group consisting of pathogenic Escherichia coli, Salmonella typhimurium, Salmonella typhi, Salmonella thyphosa, Shigella, Klebsiella, Haemophilus influenzae, Pasteurella and Actinobacillus (claim 17).

The present invention also relates to a live vaccine component containing the vaccine strain according to any of claims 1 to 17 as an active component (claim 18).

### Brief Explanation of the Drawings

Fig. 1 is a view showing a mukFEB operon and a gene product.
Fig. 2 is a view showing an α-helical coiled-coil region of a MukB protein.
Fig. 3 is a view showing a model for a MukFEB protein complex.
Fig. 4 is a view showing that a dam mutant functions as an effective vaccine strain against Salmonella typhimurium.
Fig. 5 is a view showing an example of a method for constructing a double deletion mutant of a dam gene and of a mukFEB operon according to the present invention.
Fig. 6 is a view showing the colony forming ability of the double mutant of the present invention at 22 ° C and 37 °C.

### Best Mode for Carrying out the Invention

As pathogenic bacteria of the present invention, there is no particular limitation and any bacteria can be used as long as they are pathogenic and can elicit an immune response of a host. Examples of such bacteria include pathogenic Escherichia coli, Salmonella typhimurium, Salmonella typhi, Salmonella thyphosa, Yersinia pestis, Vibrio cholerae, Clostridium perfringens, staphylococcus, Pseudomonas aeruginosa, Shigella, Klebsiella, Haemophilus influenzae, Pasteurella, Actinobacillus, Legionella, Bordetella pertussis, Francisella tularensis, Brucella, Vibrio parahaemolyticus, Neisseria gonorrhoeae, Neisseria meningitidis, Helicobacter pylori, Spirillum minus, Borrelia recurrentis, Borrelia burgdoferi, Clostridium tetani and Bacillus anthracis. Among them, bacteria which belong to gamma subclass of gram-negative Proteobacteria, in particular, bacteria having a dam gene of DNA adenine methylase, which is related to the pathogenicity-expressing function of pathogenic bacteria, and a muk gene, which is related to temperature sensitivity of pathogenic bacteria, can be used advantageously. Specific examples of these bacteria include pathogenic bacteria which invade a cell or tissue of a host such as pathogenic Escherichia coli, Salmonella typhimurium, Salmonella typhi, Salmonella typhosa, Shigella, Klebsiella, Haemophilus influenzae, Pasteurella, Actinobacillus, and the like. Further, a plant as well as an animal is exemplified as a host for pathogenic bacteria.

In the present invention, a gene mutation means a mutation of a gene carried by a pathogenic bacterium, for example, a structural gene, or a control gene such as a promoter. As variations in gene mutations, deletion mutation, frameshift mutation, amino acid substitution mutation, insertion mutation, inversion mutation and the like are exemplified. The deletion mutation here means a mutation wherein a part of DNA sequences in a chromosome or in a gene is deleted; the frameshift mutation means a kind of mutation which occurs on DNA encoding a protein wherein 3n+1 or 3n+2 (n is an integer) bases are deleted or inserted; the amino acid substitution mutation means a mutation wherein an amino acid in a protein is substituted with other amino acid by a mutation of its corresponding gene; the insertional mutation means a mutation wherein other DNA sequence is inserted into an existing DNA sequence; and the inversion mutation means a mutation wherein a part of DNA sequences in a chromosome, with its direction being inverted, is present in a chromosome, respectively.

The at least double gene mutation in the present invention means a multiple mutation wherein at least two, in other words, two or more genes possessed by the pathogenic bacteria are mutated. The preferable example of the at least double gene mutation in view of antigenicity of a vaccine strain is the one wherein a pathogenic bacterium shows at least the same growth level as that of its wild-type even though at least two genes of the pathogenic bacterium are mutated. Further, The preferable example of the at least double gene mutation in view of the safety of a vaccine strain is the one wherein the pathogenic bacteria are led to death or exhibit growth arrest or retardation even though one or more gene mutations of the at least double gene mutation are restored to normal types by reverse mutation or the like. In addition, it is preferable that at least one of the at least double gene mutation is a mutation of a gene related to the pathogenicity-expressing function of pathogenic bacteria or of a gene related to temperature sensitivity of the pathogenic bacteria, moreover, it is particularly preferable that the at least double gene mutation is a double mutation of a gene related to the pathogenicity-expressing function of pathogenic bacteria and of a gene related to temperature sensitivity of pathogenic bacteria.

As the at least double gene mutation wherein a pathogenic bacterium shows at least the same growth level as that of its wild-type even though at least two genes of the pathogenic bacterium are mutated, the mutations of two genes related to the pathogenicity-expressing function of pathogenic bacteria but not to growth inhibition of the pathogenic bacteria can be exemplified. As the at least double gene mutation wherein the pathogenic bacteria are led to death or exhibit growth arrest or retardation even though one or more gene mutations of the at least double gene mutation are restored to normal types by reverse mutation or the like, mutations of two genes are exemplified: one is a gene related to the pathogenicity-expressing function of pathogenic bacteria; and the other is a gene that can lead pathogenic bacteria to death or either arrest or retard the growth of the pathogenic bacteria subject to the restoration of the gene mutation to a normal type, for example, a gene related to temperature sensitivity in colony formation, or the like.

Examples of the gene related to the pathogenicity-expressing function of pathogenic bacteria include genes related to a toxin derived from a pathogenic bacterium such as genes encoding verotoxin, which is a protein toxin produced by pathogenic Escherichia coli 0157, Shiga-like toxin produced by Shigella, or the like; a dam gene encoding DNA adenine methylase; a gene whose expression is under control of DNA adenine methylase, for example, a gene such as spvB, pmrB, or mgtA of Salmonella typhimurium (Science 284, 967-970, 1999); and a gene encoding a protein such as SeqA that affects DNA adenine methylase activity, for instance, by delaying the methylation by DNA adenine methylase. Examples of the gene related to temperature sensitivity include a mukFEB operon being lethal to a reverse-mutated dam gene, a gene related to temperature sensitivity in colony formation of a mukF gene, a mukE gene, a mukB gene and the like that comprise the operon. It has been actually reported in U. S. A. that a dam mutant can be used as an effective vaccine strain, judging from the result of the experiments with a dam mutant of Salmonella typhimurium and a mouse for oral administration (Science 284, 967-970, 1999). According to this report, even though 10⁹ cells of dam mutant (10,000 times the LD₅₀ for a wild type strain) was administered to a mouse, the mouse survived and showed no change in its symptom, and the mouse survived after additional oral administration of 10⁹ cells of wild type strain (Fig. 4). This suggests that a dam gene can be used as a gene related to the pathogenicity-expressing function of pathogenic bacteria, but not to the growth inhibition of pathogenic bacteria.

In the present invention, at least double gene mutation of said gene related to the pathogenicity-expressing function of pathogenic bacteria, such as a dam gene, a seqA gene or the like, and of said gene related to temperature sensitivity of pathogenic bacteria such as the mukFEB operon or one or more genes selected from a mukF gene, mukE gene and mukB gene that comprise the operon, are particularly preferable. In case a vaccine strain (vaccine cell) with said combination of gene mutations is administered to a host organism, its safety as a vaccine strain will be extremely high because the vaccine strain is led to death even if the dam gene mutation of the vaccine cell is restored to a normal type by transfer of a normal dam gene or the like from other bacterium or the like by a conjugational transferable plasmid, a transducing phage or the like, or by reverse mutation or the like.

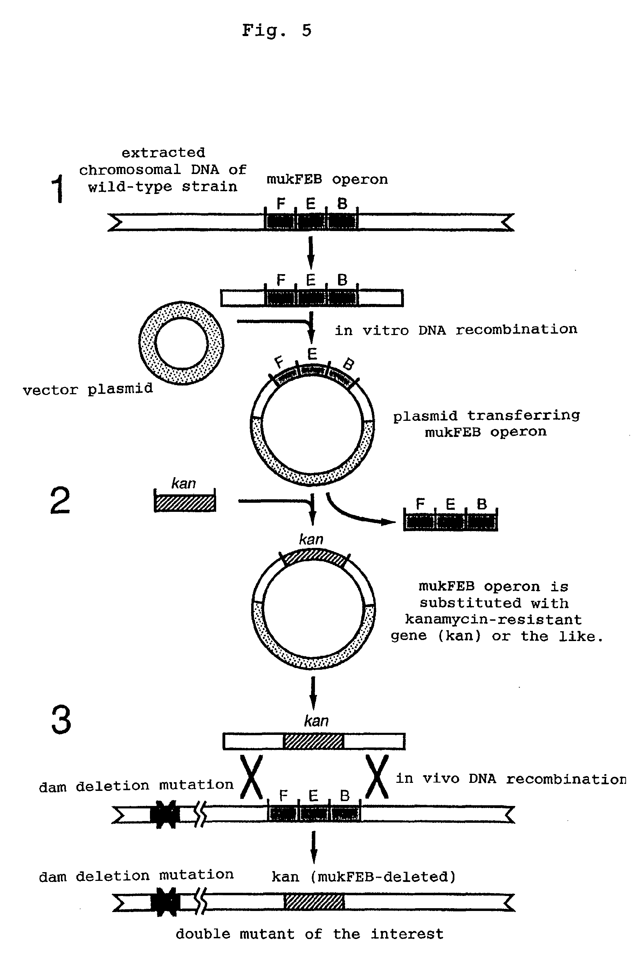

The constructing method of the at least double gene mutant is not limited in particular, and conventionally known methods of constructing a gene mutant can be used. However, it is preferable to use a constructing method of a gene mutant wherein a mutation of a target gene can be detected. It is possible to disrupt a specific gene as a target or substitute such a gene with a marker gene such as an antibiotic-resistant gene by introducing a certain DNA into a cell and selecting a homologous recombinant according to a constructing method of a gene mutant such as gene targeting (target gene recombination) or the like. For example, as shown in Fig. 5, a double gene mutant of the present invention can be constructed by selecting a homologous recombinant through the process comprising the steps of: extracting chromosomal DNA from a pathogenic bacterium; conducting PCR using the chromosomal DNA as a template and a complementary primer corresponding to the chromosomal DNA to amplify a DNA fragment containing a mukFEB operon; substituting this mukFEB operon of the amplified DNA fragment with a drug-resistant gene by a usual method; constructing a plasmid containing drug-resistant genes at both side regions of the mukFEB operon and therebetween, then preparing a linear DNA fragment containing drug-resistant genes at both side regions of the mukFEB operon and therebetween and infecting the previously described deletion mutant of a dam gene; separating a drug-resistant strain, and by confirming that it is a double mutant having deletion mutations of a dam gene (damΔ) and of a mukFEB operon (mukFEBΔ).

The vaccine strain against infection with pathogenic bacteria of the present invention is not particularly limited as long as it has at least double gene mutation and comprises pathogenic bacteria whose pathogenicity-expressing function is deficient or deteriorated, however, a vaccine strain that exhibits at least the same growth level as that of a wild type of the pathogenic bacteria, or a vaccine strain that is led to death or exhibits growth arrest or retardation in case at least one of the gene mutations is restored to a normal type is preferable. The double mutant vaccine strain of a dam mutant and of a mukFEB mutant, in particular, can be safely used because the bacterial cell is led to death even if the mutated dam gene is restored to a normal type by reverse mutation or by transfer of a dam gene from other bacteria in a host by a phage, a conjugational transferable plasmid or the like.

Said vaccine strain against infection with pathogenic bacteria can be used as a live vaccine for eliciting immune responses to pathogenic bacteria in host animals and plants, particularly animals such as human and the like, and by using the vaccine strain, a live vaccine constituent containing said vaccine strain of immunologically effective dose necessary for eliciting an immune response to pathogenic bacteria as an active component can be constructed by a usual method. Pharmaceutically acceptable vehicles, for instance, buffer liquid such as physiological saline, phosphate buffered saline (PBS), or ethanol polyols like glycerol, propyleneglycol or the like, can be used for the live vaccine component in properly diluted quantities. These live vaccine constituents are useful for completely preventing not only human but also livestock or the like from infection with pathogenic bacteria in consideration that they are extremely safer and have more potent antigenicity than conventional vaccines.

The present invention is explained more specifically with reference to examples, however, the technical scope of the present invention is not limited to these examples.

### Example 1

### (Construction of a double mutant)

Chromosomal DNA was extracted from Escherichia coli K-12 strain which belongs to gamma subclass of Proteobacteria, and 8.5 kb of BamHI DNA fragment containing a mukFEB operon was cloned on a vector plasmid pACYC184. DNA fragment (1.4 kb of HaeII DNA fragment) of a kanamycin-resistant gene (kan) was inserted instead of XhoI fragment of the mukFEB operon (containing a part of mukF, mukE and mukB). The kan-inserted mukFEB DNA fragment was excised by BamHI, and was inserted into plasmid pKH5002 (Δori, rpsL⁺, Ap^{r}) that could proliferate only in an RNaseH-deficient strain. After proliferating the resulted plasmid in an RNaseH-deficient strain, plasmid DNA was extracted and introduced into Escherichia coli MC1061 strain (rpsL mutation, Sm^{r}) by electric shock. First, a strain resistant to two drugs, kanamycin and ampicillin, was separated at 22°C, and then, after this strain was once proliferated, a strain resistant to two drugs, kanamycin and streptomycin, was separated at 22° C. It was confirmed by Southern blot analysis that a mukFEB operon on a chromosome of this strain was substituted with ΔmukFEB::kan.

In order to construct the double mutant of the present invention, P1 phages which had been proliferated in said Δ mukFEB::kan strain was infected with a dam::Tn9 strain (Cm^{r} transposon) to separate a transductant resistant to kanamycin and chloramphenicol at 22°C. The following steps were taken for confirming that this transductant was the double mutant of the interest. First, the transductant was revealed to be dam-deficient by immunofluorescence microscopy that showed the distribution of SeqA proteins throughout the cell (Mol. Cell 1, 381-387, 1998). Next, the transductant was subjected to DAPI staining and observed by an immunofluorescence microscope that it released anucleate cells at 42° C, and confirmed to be mukFEB operon-deficient.

The double mutant of Escherichia coli K-12 having deletion mutations of a dam gene (damΔ) and a mukFEB operon (mukFEBΔ) was deposited under accession number ME 9055 to National Institute of Genetics, 1111, Yata, Mishima City, Shizuoka Prefecture, on December 20, 1999, and deposited again under accession number FERM BP-7045 to National Institute of Bioscience and Human-Technology, Agency of Industrial Science and Technology M.I.T.I., 1-3, Higashi 1-chome, Tsukuba city, Ibaraki prefecture, on February 23, 2000. Each double mutant having deletion mutations of a dam gene (damΔ) and mukF, mukE or mukB was constructed in a same manner.

### (The effect of muk mutation in colony formation of each mutant)

The colony forming ability at 22°C and 37°C of the constructed 4 double mutants (damΔ and mukFEBΔ/damΔ and mukF Δ/damΔ and mukΔ/damΔ and mukBΔ), 4 kinds of strain wherein a dam gene is normal and only muk is mutated (dam⁺ and mukFEB Δ/dam⁺ and mukFΔ/dam⁺ and mukEΔ/dam⁺ and mukBΔ), a strain wherein muk genes are normal and only dam is mutated (damΔ and mukFEB⁺), and a wild-type strain (dam⁺ and mukFEB⁺) were examined. Each strain was seeded on a plate containing M9 synthetic medium (Molecular Cloning, Second edition, Vol. 3, Appendixes A. 2, Cold Spring Harbor Laboratory, N. Y., 1989) supplemented with 0.2 mM magnesium sulfate, 0.1 mM calcium chloride, glucose (0.5 %), Difco Casamino acids (0.4 %), L-tryptophan (50 µg/ml) and agar (1.5 %)(hereinafter called "MCAT agar medium"), and cultured for 2 days at 22°C or for 20 hours at 37°C. The results are shown in Table 1. These results are shown with colony forming ability at 22°C as an index of 1. These results suggest that the double mutant of damΔ and of mukFEBΔ forms a colony on the agar medium even at 37°C, but colony forming ability of the mukFEBΔ mutant having a normal dam gene is reduced to about one ten thousandth (~10⁻⁴), and the strain is found to be unable to form a colony at 37°C. This indicates that when the double mutant of damΔ and of mukFEBΔ is used as a vaccine strain, the vaccine cells will be led to death even though a normal dam gene is transferred to restore the mutated dam gene to a normal type in vivo. In other words, it is revealed to be extremely safe as a vaccine strain. In deletion mutants having deletion of a mukF, a mukE or a mukB gene respectively in stead of a mukFEB operon, the same results were obtained as the case using mukFEB deletion mutants as shown in Table 1.

**Table 1**

| Bacterial strains | Colony forming ability ^{n.b.)} | |
|---|---|---|
| | 22° C | 37° C |
| Wild type (dam⁺ mukFEB⁺) | 1 | 1 |
| damΔ mukFEB⁺ | 1 | 1 |
| damΔ mukFΔ | 1 | 1 |
| damΔ mukEΔ | 1 | 1 |
| damΔ mukBΔ | 1 | 1 |
| damΔ mukFEBΔ | 1 | 1 |
| dam⁺ mukFΔ | 1 | ⁻10⁻⁴ |
| dam⁺ mukEΔ | 1 | ⁻10⁻⁴ |
| dam⁺ mukBΔ | 1 | ⁻10⁻⁴ |
| dam⁺ mukFEBΔ | 1 | ⁻10⁻⁴ |
| n.b.: Colony forming ability at 22°C is indicated as 1. Colonies were formed on MCAT agar media. Incubated at 22°C for 2 days. Incubated at 37°C for 20 hours. Normal genes are shown with +. Genes with Δ indicate deleted genes. | | |

Followings are indicated by these results: the transcription/expression of various genes is remarkably increased when using the double mutant vaccine strain of dam Δ and mukFEBΔ; if the double mutant vaccine strain is orally administered to a host animal, although the strain forms a colony on a mucous membrane, it does not proliferate and has no colony forming ability in deep parts of organ tissues, and exhibits nonpathogenicity; as said double mutant exhibits far more potent antigenicity than a pathogenic wild type, a host animal having been administered with said double mutant acquires potent immunity to this bacterium, in other words, even if the host animal is infected with a pathogenic strain later, its immune function prevents the animal from onset of a disease by inhibiting proliferation of the strain; it is sufficiently promising that even though reverse mutation occurs or a DNA adenine methylase gene is transferred from other bacterium by a phage, a conjugational transferable plasmid or the like in a host, the bacterial cell will be led to death.

### Example 2

### (Construction of a double mutant)

Bacterial strains used in example 2 are shown in Table 2. As to preparation methods of media and bacteria, methods previously described (J. Bacteriol. 171: 1496-1505, 1989; Mol. Gen. Genet. 250: 241-251, 1996) are used.

**Table 2**

| Bacterial strains | Genotypes | References |
|---|---|---|
| YK1100 | W3110 except trpC9941 | Yamanaka et al., 1996 |
| AZ5372 | YK1100 except ΔmukB::kan | Yamanaka et al., 1996 |
| AZ5450 | YK1100 except mukE::kan | Yamanaka et al., 1996 |
| AZ5381 | YK1100 except mukF::kan | Yamanaka et al., 1996 |
| OT7 | PB103 except ΔmukFEB::kan | Yamazoe et al., 1999 |
| NK7253 | NK7254 except seqA::tet | Lu et al., 1994 |
| KA468 | NH5402-1 except dam-13::Tn9 | T. Katayama |

According to the method previously described (Experiments With Gene Fusions, Cold Spring Harbor Laboratory, N.Y., 113-117, 1984), transduction of defective dam and/or seqA genes was performed with phage P1 vir. Each cell of the wild-type strain YK1100 and mukB deletion mutant AZ5372, mukE deletion mutant AZ5450, mukF deletion mutant AZ5381, mukFEB operon deletion mutant OT7 were infected with phage P1 vir lysate obtained from KA468 strain having a defective dam::cat gene. Subsequently, for inhibiting reinfection through phage P1 vir, the cells were incubated for 5 days at 22°C on L agar medium containing chloramphenicol (7 µg/ml) and sodium citrate (20 mM), after which chloramphenicol-resistant transductants were isolated. After isolating colonies of 10 transductants on L agar plate containing sodium citrate at 22° C once, these transductants were confirmed to be dam mutants by observing SeqA localization by immunofluorescence microscopy using an anti-SeqA antibody. Double mutants of dam and muk, KK266, KK267, KK269, KK268 and KK270 have been thus prepared, and confirmed to be dam deletion mutants by immunofluorescence microscopy.

Similarly, by P1 transduction using phage P1 vir lysate obtained from NK 7253 strain having a defective seqA::tet gene, the defective seqA::tet gene was introduced into each cell of a wild-type strain YK1100 and a mukB deletion mutant AZ5372, a mukE deletion mutant AZ5450, a mukF deletion mutant AZ5381, a mukFEB operon deletion mutant OT7 , and each cell of said double mutants, KK266, KK267, KK269 and KK268. Then the cells were incubated for 5 days at 22°C in L agar plates containing tetracycline (7 µg/ml) and sodium citrate (20 mM), followed by isolation of tetracycline-resistant transductants. After the isolation, the obtained transductants were confirmed to be seqA mutants by immunofluorescence microscopy for examining whether a SeqA protein was present in the transductants. Double mutants of seqA and muk. KK259, KK248, KK250, KK251 and KK254, and triple mutants of dam, seqA and muk, KK278, KK2779, KK281, KK280 and KK282 have been thus prepared.

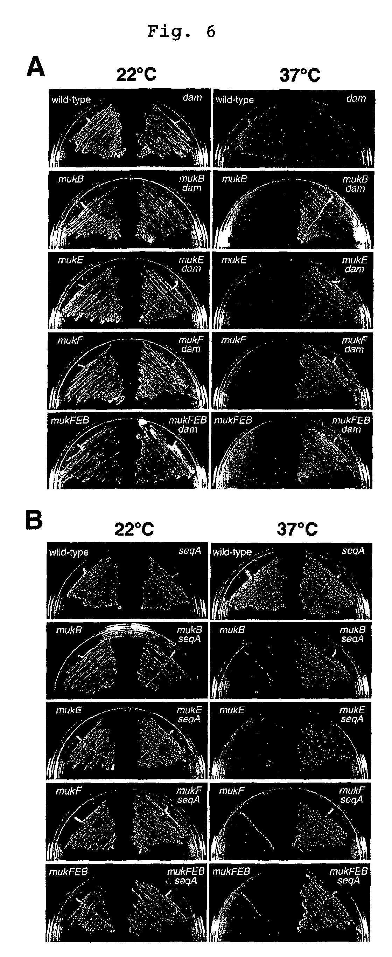

Next, colony forming ability at each temperature of 22° C, 30° C, 37° C and 42° C was examined with regard to each of the wild type strain, the dam mutant, the seqA mutant, the muk mutant, the double mutant of dam and muk, the double mutant of seqA and muk, and the triple mutant of dam, seqA and muk. As media, the MCAT agar medium and L agar medium (1 % Difco tryptone peptone, 0.5 % Difco yeast extract, 0.5 % NaCl, 1.5 % agar; pH7.4) were used. Each bacterial strain was proliferated at 22°C until logarithmic growth phase in MCAT medium and L medium without agar, and diluted with physiological saline, then seeded onto each of MCAT agar and L agar plates. These plates were incubated for 1 to 4 days at each temperature of 22° C, 30° C, 37° C and 42° C, until colony formations could be observed. The results are shown in Table 3 and Fig. 6. The values in Table 3 are shown in comparison to colony forming ability at 22°C, which is indicated as 1.

**Table 3**

| Bacterial strains | Related genotypes | Colony forming ability | | | | | | | |
|---|---|---|---|---|---|---|---|---|---|
| | | MCAT medium | | | | L medium | | | |
| | | 22°C | 30°C | 37°C | 42°C | 22°C | 30°C | 37°C | 42°C |
| YK1100 | Wild type | ≡1 | 0.8 | 1.0 | 0.9 | ≡1 | 0.7 | 3.1 | 1.9 |
| KK266 | dam | ≡1 | 1.0 | 0.8 | 1.1 | ≡1 | 0.9 | 1.0 | 0.7 |
| KK259 | seqA | ≡1 | 1.0 | 0.9 | 1.1 | ≡1 | 1.2 | 0.9 | 0.6 |
| KK278 | dam seqA | ≡1 | 0.9 | 1.2 | 1.2 | ≡1 | 2.2 | 1.5 | 1.7 |
| | | | | | | | | | |
| AZ5372 | mukB | ≡1 | 0.8 | 2×10⁻⁴ | <1×10⁻⁵ | ≡1 | 0.3 | <8×10⁻⁴ | <1×10⁻⁵ |
| KK267 | mukB dam | ≡1 | 1.3 | 0.7 | <1×10⁻⁵ | ≡1 | 1.0 | 0.4 | <1×10⁻⁵ |
| KK248 | mukB | ≡1 | 1.0 | 0.8 | <1×10⁻⁵ | ≡1 | 1.1 | 1.0 | <1×10⁻⁵ |
| | seqA | | | | | | | | |
| KK279 | mukB dam seqA | ≡1 | 1.1 | 0.2 | <1×10⁻⁵ | ≡1 | 1.0 | 1.4 | <1×10⁻⁵ |
| | | | | | | | | | |
| AZ5450 | mukE | ≡1 | 0.8 | <5×10⁻⁴ | <1×10⁻⁵ | ≡1 | 1.0 | <2×10⁻⁴ | <1×10⁻⁵ |
| KK269 | mukE dam | ≡1 | 0.9 | 0.6 | <1×10⁻⁵ | ≡1 | 2.6 | 1.8 | <1×10⁻⁵ |
| KK250 | mukE seqA | ≡1 | 1.0 | 1.0 | <1×10⁻⁵ | ≡1 | 0.8 | 0.1 | <1×10⁻⁵ |
| KK281 | mukE dam seqA | ≡1 | 0.9 | 0.1 | <1×10⁻⁵ | ≡1 | 1.0 | <4×10⁻³ | <1×10⁻⁵ |
| | | | | | | | | | |
| AZ5381 | mukF | ≡1 | 1.0 | <1×10⁻⁴ | <1×10⁻⁵ | ≡1 | 0.9 | <4×10⁻⁴ | <1×10⁻⁵ |
| KK268 | mukF dam | ≡1 | 0.9 | 0.7 | <1×10⁻⁵ | ≡1 | 1.1 | 1.0 | <1×10⁻⁵ |
| KK251 | mukF seqA | ≡1 | 1.5 | 0.9 | <1×10⁻⁵ | ≡1 | 1.0 | 0.3 | <1×10⁻⁵ |
| KK280 | mukF dam seqA | ≡1 | 0.9 | 0.1 | <1×10⁻⁵ | ≡1 | 0.9 | <3×10⁻³ | <1×10⁻⁵ |
| | | | | | | | | | |
| OT7 | mukFEB | ≡1 | 1.0 | <1×10⁻⁴ | <1×10⁻⁵ | ≡1 | 0.8 | <5×10⁻⁴ | <1×10⁻⁵ |
| KK270 | mukFEB dam | ≡1 | 1.1 | 0.8 | <1×10⁻⁵ | ≡1 | 0.8 | 0.7 | <1×10⁻⁵ |
| KK254 | mukFEB seqA | ≡1 | 1.1 | 1.0 | <1×10⁻⁵ | ≡1 | 0.5 | 0.7 | <1×10⁻⁵ |
| KK282 | mukFEB dam seqA | ≡1 | 1.5 | 0.7 | <1×10⁻⁵ | ≡1 | 1.0 | 6×10⁻³ | <1×10⁻⁵ |

The double mutants of dam and muk, and of seqA and muk, and the triple mutant of dam, seqA and muk, which were incubated on MCAT agar media at 22°C, exhibited the same level of colony forming ability as that of the wild type, the dam mutants strain, the seqA mutant and the muk mutant. When incubated at 37°C, although each muk deletion mutant having a wild-type dam gene could not form colonies, the double mutants of dam and muk, and of seqA and muk, and the triple mutant of dam, seqA and muk showed colony forming ability even at 37°C. The muk gene-deficient strains could not survive at 42° C. When L agar media were used, almost the same results were obtained as in the cases using MCAT agar media. These results indicate that when the double mutants of dam and muk, and of seqA and muk, and the triple mutant of dam, seqA and muk are used as a vaccine strain, even if normal dam genes and/or seqA genes are transferred in vivo and the mutated dam genes and/or seqA genes are restored to normal types, this vaccine cell is led to death. In other words, they are revealed to be extremely safe as a vaccine strain.

### Industrial Applicability

The vaccine strain of the present invention, such as the double mutant of a dam gene and a mukFEB operon or the like, is useful as a practical live vaccine because it is extremely safer than conventional strains while maintaining its potent antigenicity, and can be used as effective means to completely prevent human, livestock and the like from infection with pathogenic bacteria.

## Claims

1. A vaccine strain against infection with pathogenic bacteria being comprised of pathogenic bacteria having at least double gene mutation and whose pathogenicity-expressing function is deficient or deteriorated.

2. The vaccine strain against infection with pathogenic bacteria according to claim 1, wherein the pathogenic bacteria having at least double gene mutation and whose pathogenicity-expressing function is deficient or deteriorated exhibit at least the same growth level as that of wild types of the bacteria.

3. The vaccine strain against infection with pathogenic bacteria according to claim 1, wherein when one or more gene mutations of the at least double gene mutation are restored to normal types, the pathogenic bacteria are led to death or exhibit growth arrest or retardation.

4. The vaccine strain against infection with pathogenic bacteria according to any of claims 1 to 3, wherein at least one of the at least double gene mutation is a mutation of a gene related to the pathogenicity-expressing function of pathogenic bacteria.

5. The vaccine strain against infection with pathogenic bacteria according to any of claims 1 to 4, wherein at least one of the at least double gene mutation is a mutation of a gene related to temperature sensitivity of pathogenic bacteria.

6. The vaccine strain against infection with pathogenic bacteria according to any of claims 1 to 3, wherein the at least double gene mutation is a double mutation of a gene related to the pathogenicity-expressing function of pathogenic bacteria and of a gene related to temperature sensitivity of pathogenic bacteria.

7. The vaccine strain against infection with pathogenic bacteria according to claim 4 or 6, wherein the gene related to the pathogenicity-expressing function is related to a toxin derived from a pathogenic bacterium.

8. The vaccine strain against infection with pathogenic bacteria according to claim 4 or 6, wherein the gene related to the pathogenicity-expressing function encodes DNA adenine methylase.

9. The vaccine strain against infection with pathogenic bacteria according to claim 5 or 6, wherein the gene related to temperature sensitivity is a mukFEB operon.

10. The vaccine strain against infection with pathogenic bacteria according to claim 5 or 6, wherein the gene related to temperature sensitivity is one or more genes selected from a mukF gene, a mukE gene and a mukB gene.

11. The vaccine strain against infection with pathogenic bacteria according to any of claims 1 to 3, wherein the at least double gene mutation is a double mutation of a gene encoding DNA adenine methylase and of a mukFEB operon.

12. The vaccine strain against infection with pathogenic bacteria according to any of claims 1 to 3, wherein the at least double gene mutation is a double mutation of a gene encoding DNA adenine methylase and of one or more genes selected from a mukF gene, a mukE gene and a mukB gene.

13. The vaccine strain against infection with pathogenic bacteria according to any of claims 1 to 3, wherein the at least double gene mutation is a triple mutation of a gene encoding DNA adenine methylase, a gene encoding Seq A, and a mukFEB operon.

14. The vaccine strain against infection with pathogenic bacteria according to any of claims 1 to 3, wherein the at least double gene mutation is a triple mutation of a gene encoding DNA adenine methylase, a gene encoding Seq A, and one or more genes selected from a mukF gene, a mukE gene and a mukB gene.

15. The vaccine strain against infection with pathogenic bacteria according to any of claims 1 to 14, wherein the gene mutation is one or more gene mutations selected from deletion mutation, frameshift mutation, amino acid substitution mutation, insertional mutation and inversion mutation.

16. The vaccine strain against infection with pathogenic bacteria according to any of claims 1 to 15, wherein the pathogenic bacteria invade a cell or tissue of a host.

17. The vaccine strain against infection with pathogenic bacteria according to claims 16, wherein the pathogenic bacteria invading a cell or tissue of a host is selected from a group consisting of pathogenic Escherichia coli, Salmonella typhimurium, Salmonella typhi, Salmonella thyphosa, Shigella, Klebsiella, Haemophilus influenzae, Pasteurella and Actinobacillus.

18. A live vaccine component containing the vaccine strain according to any of claims 1 to 17 as an active component.
